# EUROPEAN PATENT APPLICATION

(11) **EP 1 868 123 A1**
(43) Date of publication of application: **19.12.2007**
(21) Application number: 06115481.1
(22) Date of filing: 14.06.2006
(51) Int. Cl.: G06F 19/00, A61B 5/00, A61B 5/117, A61G 12/00, G08B 5/22

(54) **Patient monitor with subdued alarm in presence of caregivers**

(71) Applicant: The General Electric Company, Schenectady NY 12345 (US)
(72) Inventor: Laakonen, Marko, 01660, VANTAA (FI)
(74) Representative: Laako, Tero Jussi

(57) **Abstract**

The invention concerns a system for electronic alarm control of patient monitors (10) in the presence of caregiving person(s). For this purpose each caregiving person (1) carries an identification badge (11), which include e.g. a tag (4) storing a personal ID-data (D_{C}). The patient monitor comprises sensors (3a, 3b, 3c...) for said medical data (D_{M}), a processor (15), a display (16), and an operator interface (7) with an alarming unit (17), and a data transfer unit (8) capable to read the data contents of said badges within a limited reading space (V) inside or around or beside said patient monitor. The alarming unit (17) is controlled by said processor so that an alarm is activated when said medical data measured from the patient deviates from preset value(s), and the activated alarm is subdued when at least one of said caregiving person badges enters said reading space within/around/beside said patient monitor, and is detected by said data transfer unit using the data from the badge (11).

## Description

The invention concerns a system for electronic alarm control of patient monitors in the presence of nursing staff

Patient monitoring systems are commonly used for monitoring the condition of a patient, such as in coronary care units and intensive care units of a hospital. Such systems typically include a bedside monitor having one or more sensors, such as ECG sensors, blood pressure sensors and temperature sensors, attached to the patient. The sensors measure various physiological parameters of the patient. The measured parameters are processed by a processor. The processed information may be shown on a display and stored for later analysis. Patient physiological information from several bedside monitors may be forwarded to a central station located, for example, at a nursing station. The bedside patient monitor and the central station may display physiological parameters as waveforms and/or numerical values. Another important function of patient monitoring systems is to generate alarms when one or more of the physiological parameters indicate that the patient requires attention. Such alarms are necessary because it is not feasible for the display screen of the patient monitoring system to be observed continuously. Alarms are typically annunciated both visibly and audibly.

Patent publication US 5,464,012 discloses a method and apparatus for detecting an alarm in a patient monitoring system during the transient period, which occurs after performing a procedure that intentionally alters the patient's state. The patient monitoring system executes the steps of initiating a target mode when the user sets target limits of a physiological parameter, establishing dynamic limits that vary with time, comparing measured parameter values with the dynamic limits, and generating an alarm when any of the measured parameter values falls outside the dynamic limits. The target mode is terminated when any of the measured parameter values falls within the target limits. The dynamic limits ensure that the physiological parameter is monitored during the transient period after an intervention procedure, without generating false alarms. The aim in this method and apparatus is to accommodate the situation where a clinician intentionally alters the state of a patient, such as by administering an anaesthetic or a drug, whereupon the steady state parameter values are not proper causing non-wanted alarming.

A health care facility management system is available from Versus Technology, Inc. The system employs IR/RF technology to provide real-time, continuous, location-specific information about people and equipment as they move through the facility. Each person or piece of equipment wears a passive transmitting badge with a unique ID. Data is collected to provide information on room status, equipment being utilized for a given patient, and presence and frequency of interaction between patient and staff. Patient movement is facilitated by directing patients to available testing areas. Instant knowledge of the onset and duration of a procedure allows the facility to plan ahead. The amount of time spent between a patient and a caregiver is recorded, as the amount of time for a particular procedure. The data is used for reports, particularly those for compliance with JCAHO standards. Some of the reports available include a "Tracking Log", which details the movement of an individual or piece of equipment throughout the facility, including identifying each room entered, arrival and departure time and total time spent in each room. A 'Time Together' report shows how much time different people or equipment has spent together in a particular room for any given time period. This data may be used for billing or audit reports. Although the mechanism of the time together report is not set out, it appears that it searches for same-room presence during a common time frame for two people/equipment, rather than recording a direct proximity signal between the two. <http://www.versustech.com>.

Also patent publication US 6,154,139 discloses this kind of method and system for locating subjects within a tracking environment, such as a health care facility. For this purpose the personnel, such as patients and caregivers, are provided with transmitting tags, which transmit both an IR - line-of-sight - identifying signal and a RF - non-line-of-sight - identifying signal. The IR signal is effective in accurately determining location to a specific degree. However, because it requires line-of-sight, it cannot be used to locate personnel in sensitive areas where IR receivers are not placed. In this case, a RF signal may reach a RF receiver within a certain distance, even through walls. Therefore, if a patient presses a distress call from the bathroom, the RF receiver transmits this signal to the central processor, which can locate the patient by way of the last IR signal received, e.g. hallway outside of bathroom.

Linked interaction between two objects, for example a person and equipment within facility is taught by Axcess Inc. Using active RFID tagging technology, whereupon the stationary instrumentation uses tag activators and receivers with antenna, and the tags includes battery and transmitter, a system provides for tracking and location assets throughout a facility on-demand, determining equipment status and inventory, locating personnel, protecting assets from unauthorized removal from a ward or facility. The latter is achieved by providing tags for each piece of equipment. As the equipment approaches an exit door or other restricted area, the tag is identified and appropriate alarm signal can be sent. Personnel tags can be linked via software to particular assets or a certain type of equipment, with a defined relationship permitting the free movement of the equipment only when it is accompanied by an authorized person. The system is programmed to override the alarm signal when the identified location of a piece of equipment and an authorized person coincide. This system can be used for asset management, personnel and vehicle access control, personnel monitoring, production and process control, and inventory tracking. <http://www.axcessinc.com>.

It is the object of the invention to have a system, which enable entering of a person or persons to take care of a patient without annoyance of especially the audible alarm signal from the patient monitor, which has been switched to this alarming state because of measured deviation(s) from determined optimum medical parameters for this patient. A second object of the invention is to have this kind of system, which enable avoidance of the annoyance of the audible alarm signal without special measures from said person or persons, whereupon they can make the necessary operations or actions to the patient without any delay. A further object of the invention is to have this kind of system, which automatically controls that only authorized members of the nursing staff are able to do the actions without said annoyance, and make such changes to the settings in the system, which concern the patient in question.

The above-defined objects can be achieved by means of a system defined in claims.

According to the first aspect of the invention, it is provided a system for electronic alarm control of patient monitors in the presence of caregiving person or persons, comprising: each caregiving person carries an identification badge; said identification badge includes at least a tag storing a personal ID-data in a tag memory, or at least a marking with a personal ID-data on said badge; said patient monitor comprises: sensors attached to a patient and measuring medical data from the patient, a processor, a display, and an operator interface with an alarming unit, a data transfer unit capable to read the data contents of said personal tags within a limited reading space inside or around or beside said patient monitor, said sensors, said operator interface, and said data transfer unit are connected to said processor, which is operating with a predetermined program and/or logics; said alarming unit being controlled by said processor so that: an alarm is activated when said medical data measured from the patient deviates from a preset value or values or range of values, the activated alarm is subdued when at least one of said caregiving person badges enters said reading space within/around/beside said patient monitor, and is detected by said data transfer unit using the data from the tag memory or onfrom the badge.

According to the second aspect of the invention, it is provided a system for electronic alarm control of patient monitors in the presence of caregiving person or persons comprising: each caregiving person carries an identification badge; said identification badge includes at least a tag storing a personal ID-data in a tag memory, or at least a marking with a personal ID-data on said badge; said patient monitor comprises: sensors attached to a patient and measuring medical data from the patient, a processor, a display, and an operator interface with an alarming unit, a system memory, in which at least said ID-data is stored, or a data connection to a data network of the facility in question, which network comprises a system memory storing at least said ID-data, a data transfer unit capable to read the data contents of said personal tags within a limited reading space inside or around or beside said patient monitor, said sensors, said memory, said operator interface, and said data transfer unit are connected to said processor, which is operating with a predetermined program and/or logics; said alarming unit being controlled by said processor so that: an alarm is activated when said medical data measured from the patient deviates from a preset value or values or range of values, the activated alarm is subdued when at least one of said caregiving person badges enters said reading space within/around/beside said patient monitor, and is detected by said data transfer unit using the data from the tag memory or onfrom the badge, and data from the system memory.

There are, accordingly, two alternatives, the necessary data can be stored on or in the badges only, or a portion of the data can be stored in he system m memory or memories.

The invention is now described with reference made to the accompanying drawings.

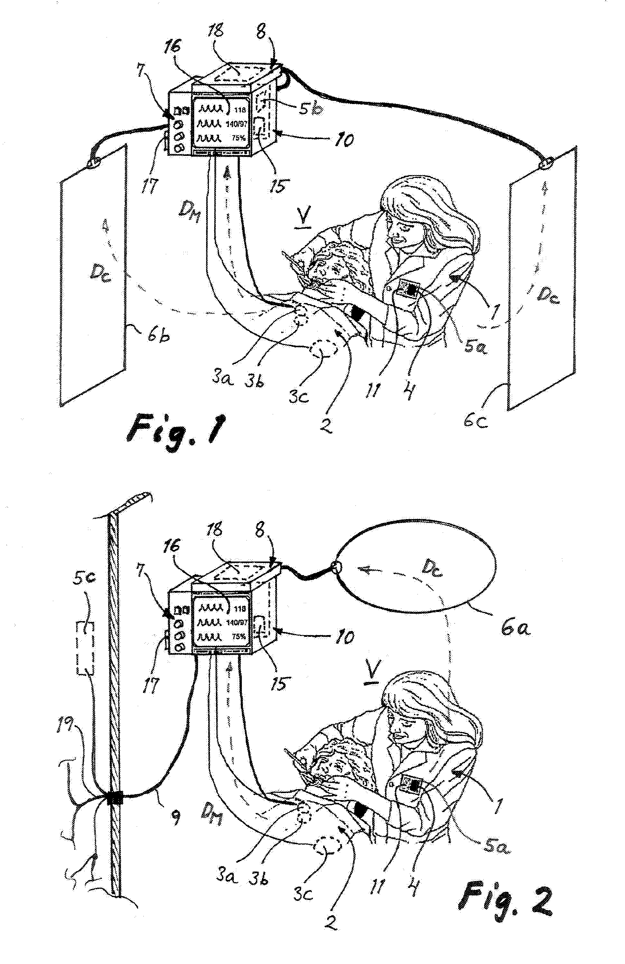

Fig. 1 shows the first preferred embodiment of the system according to the invention with a set of two antennas in an independent patient monitor for reading the RFID-tag of nursing staff member, schematically in an axonometric view.

Fig. 2 shows the second preferred embodiment of the according to the invention with one antenna in a patient monitor connected to a data network of a hospital or medical centre for reading the RFID-tag of nursing staff member, schematically in an axonometric view.

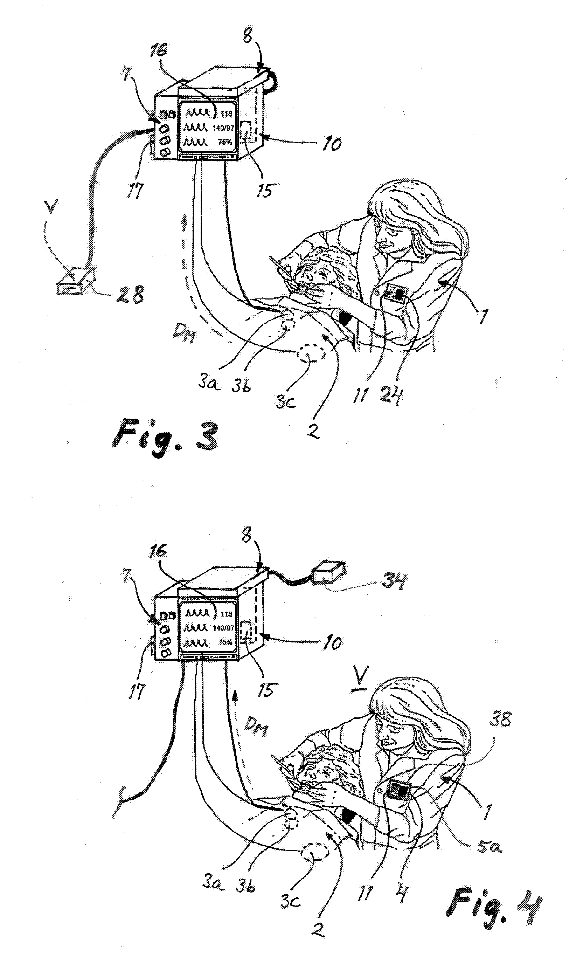

Fig. 3 shows the third embodiment of the system according to the invention with a card reader in an independent patient monitor for reading the marking(s) on the badge of the nursing staff member, schematically in an axonometric view.

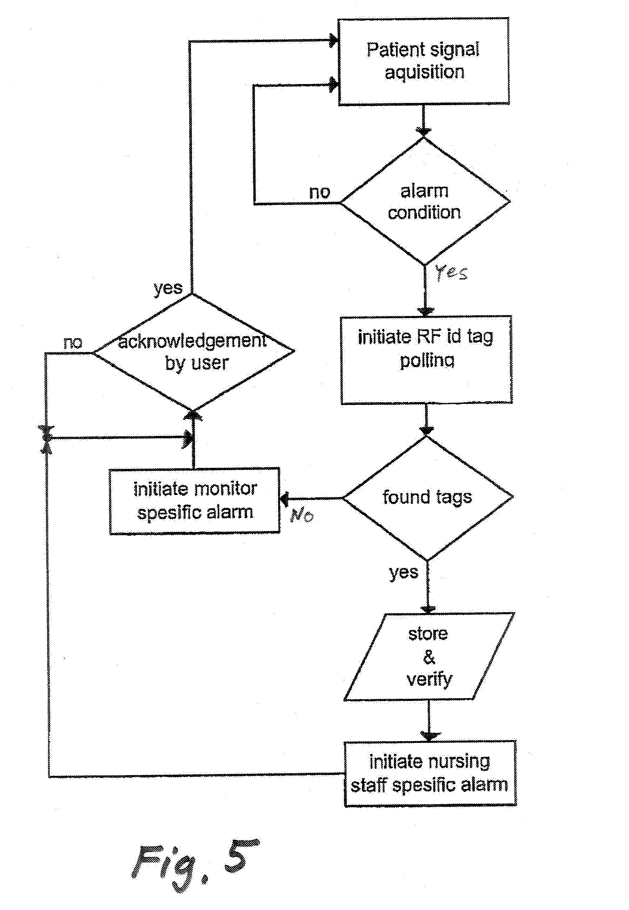

Fig. 4 shows the fourth embodiment of the system according to the invention with a receiver in an optionally independent patient monitor for reading the data sent by a transmitter in the badge of the nursing staff member, schematically in an axonometric view.

Fig. 5 shows one possible chain of program steps or logic steps according to the invention.

At first it is explained the preferred embodiment of the invention, and thereafter some of the alternative solutions to attain the objects of the invention.

The system for electronic alarm control of patient monitors 10 in the presence of nursing staff concerns, if possible, among other things the competences or qualifications among the caregiving persons 1 of said nursing staff, e.g. physicians or nurses. Each physician or nurse etc. has gone through different learning and training of different levels, have different experience, and accordingly have different competence or qualification also within their own professional field, and of course as compared to different professions. Accordingly, to each member 1 of the nursing staff an authorization to perform specified medical operation or operations can be and must be determined, which authorization is in line with their competence. In many fields there is also legal regulations, which shall be taken account in facilities like hospitals, clinics, and medical centers. Anyway, in the facilities there is a plurality of tasks, i.e. specified medical operation or operations, which can be performed in the facility in question, because the facility have proper instrumentation and qualified personnel for those operations. Each of the specified medical operation or operations requires a predetermined competence and authorization from at least one member 1 of the nursing staff, which required competence/authorization for each specified medical operation is defined, which is normal decision making, and registered. According to the invention this registering of the predetermined competence and authorization to perform specified medical operation or operations is stored: In the first embodiment of the invention in the memory 5a of tag 4 attached in the badge 11 for the person or member 1 of the nursing staff; Or in the second embodiment of the invention either in the system memory 5b of the patient monitor 10, or in the system memory 5c of the data network 19 of the facility. The mentioned competence/authorization data, and said data specifying the allowed medical operation(s), as well as the optional personal ID-data D_{c} of every member of the nursing staff are stored so linked to each other that the competence/authorization data, medical operation(s) data and ID-data of each person 1 can be utilized together in the processor 15. If the competence/authorization data is stored in the tag memory 5a, that data can be forwarded, after reading thereof from the tag with the Data transfer unit, to the processor 15 in a straightforward manner. In this case the tag 4 in a way says that the person present is qualified to perform operation A, B, C" - in reality it is question about a data code. If the competence/authorization data is stored in one of the system memories 5b or 5c, the identification data, i.e. ID-data read from the tag with the Data transfer unit, shall be compared with the competence/authorization data, which is connected to the ID-data of the personnel, receivable from the system memory 5b, 5c, and after this comparison the valid competence/authorization data can be forwarded to the processor 15. This kind of data processing and linking is known technology, and is not explained more in detail here.

Each caregiving person or member 1 of the nursing staff in the facility, like hospital or clinic or medical center, carries an identification badge 11, which includes at least a short-range radio communication tag 4 storing either at least a personal electronic competence/authorization data D_{c} in a tag memory 5a, or alternatively at least a personal electronic ID-data D_{c} in a tag memory 5a. The identification badge 11 can, of course, also include a photograph of the person 1, his/hers name, his/hers position in the organization, his/hers competence/authorization etc. in a form that a human being can directly read. This kind of data can also be used for identification of the member 1 of the nursing staff. In fact the necessity to carry some kind of identification badge is standard practice in many places today, but the short-range radio communication tag 4 storing the personal competence/authorization data, or the personal ID-data otherwise on the badge 11 is the feature that is utilized according to the invention. Preferably the short-range radio frequency communication tag 4 is a passive transponder, which typically comprises a coil antenna and a resonance circuit. For the invention such passive transponders 4 are used, which further comprise a semiconductor chip with at least a tag memory 5a. These kinds of transponders are commercially available from many manufacturers, and accordingly, they are not explained in detail. It is also possible to use active tags, whereupon the badges have radio frequency or infrared frequency or some other frequency transmitter to send the personal data D_{C} to the patient monitor. Also card readers, which need contact, or which work without contact can be used. The mentioned personal data D_{C} can be in any form useful for this purpose. Accordingly, various coding systems and data processing logics or programs are not explained. Already standard transponders with a semiconductor chip normally include a free memory of 256 bits, which allow good security for identification and authentication. If necessary it can be used transponders that have larger size of memory, so that all possible data, whether concerning ID-data and/or competence/authorization data and/or any other data, which is considered necessary, can be stored in the tag memory 5a.

The patient monitor 10, which definition here means the whole device used for the invention, comprises sensors 3a, 3b, 3c... that are attached to a patient 2 and are measuring medical data D_{M} from the patient, a processor 15 like those used in computers to process the data received, a display 16 that show the selected information to the personnel, and an operator interface 7 with an alarming unit 17, through which interface the patient monitor 10 can be controlled, its setting altered etc. The patient monitor 10 also comprises a system memory 5b and/or a data connection 9 to a data network 19 of the facility in question, which network comprises system memory or memories 5c. In case of the first embodiment of the invention, whereupon the tag memory 5a stores all the needed competence/authorization data of the caregiving person 1 carrying it, the system memory 5b, 5c can be neglected. In spite of this the system memory or memories 5b, 5c can be used for other purposes. In case of the second embodiment of the invention, whereupon the tag memory 5a stores only a portion of the needed data for competence/authorization of the caregiving person 1 carrying it, and the system memory 5b or 5c stores the rest of the needed data for competence/authorization for the caregiving persons, all of the mentioned memories shall be included in this system. Further, the patient monitor 10 comprises a Data transfer unit 8 capable to read the data contents of said personal tags 4, and antenna 6a or antennas 6b, 6c arranged to have a limited reading space V around or beside said patient monitor. The sensors, the respective memory 5a or memories 5a and 5b, or 5a and 5c, the operator interface 7, and the Data transfer unit 8 are connected to the processor 15, which is operating with a predetermined program and/or logics. These connections and processing are electronics and computer technology, and a person skilled in the art is able to design and assemble a device and make a proper program for the processor of the device, which work according to the principles of the invention. Accordingly, the construction and programs are not explained in detail in this text.

According to the invention the alarming unit 17 is controlled by the processor 15 so that: an alarm is activated when said medical data D_{M} measured from the patient deviates from a preset value or values or range of values; and the activated alarm strength is subdued when at least one caregiving person enters said reading space V around or beside said patient monitor, and is detected by said data transfer unit. The presence of one or several caregiving persons can be detected in several ways. One alternative is that the data transfer unit 8 is a short-range radio transmitter unit in the patient monitor 10 with an antenna 6a or set of antennas 6b, 6c arranged to have a limited reading space V around or beside said patient monitor, and said tag 4 is a passive RFID tag with a tag memory 5a, which is explained above. The personal ID-data D_{c} is then a byte or bytes stored in said tag memory 5a, and the limited reading space V around the patient monitor 10 is attained by antenna 6a configuration and/or antenna 6b, 6c positioning, and by low energy of the radio frequency field. A second alternative is that said data transfer unit 8 is an optical or magnetic or electrical card reader in the patient monitor 10, whereupon the personal ID-data D_{c} is a line code, or set of characters and/or numbers, e.g. name of the person or some other personal indentification code, printed on the badge 11, or the personal ID-data D_{c} is a byte or bytes stored in the tag memory 5a in the badge 11. Here the badge or tag is passive, and the data transfer unit read the information onfrom the badge - more precisely from the surface of the badge - e.g. through laser reading system, or through a camera with a image or text detecting program in the processor, or through electrical contacts, when the person inserts the badge in the reader. The limited reading space is the card slot data transfer unit. A third alternative is that said data transfer unit 8 is a receiver unit in the patient monitor 10 together with a RF-transmitter or IR-transmitter in the badge 11 producing a limited reading space V around or beside said patient monitor, and said tag 4 is an active RFID tag or IR tag with a tag memory 5a. Here the badge is active and send the ID-data, which is a byte or bytes stored in said tag memory, through infrared radiation or radio frequency to the data transfer unit after activation of the badge. The limited reading space is attained either by antenna 6a configuration and/or antenna 6b, 6c positioning, and by low energy of the radio frequency field, or alternatively by the visibility of the infrared radiation.

Now it is question about whether some person is a caregiving person, i.e. member of a nursing staff. There are several alternatives for this. If the ID-data D_{C} is readable by the data transfer unit 8, the person can be considered to a caregiving person, whereupon persons not being caregiving person do not have such badges, but maybe different badges. If the ID-data contains a further data indicating the fact that the badge belong to a member of the nursing staff, i.e. the tag memory carries the information, is a straightforward way to see that the person is a caregiving person. Further, if the ID-data can be found from the system memory 5b or 5c as such, the person is a caregiving person.

The program and/or logics of the processor 15 is such that it allows the activated alarm to be switched off through the operator interface 7 only by a detected caregiving person, preferably only by such person, who has checked to have such authorization that enable him/her to perform the actions necessitated by the measured medical data having caused said alarm. In this case the activated alarm can be also restored to the initial high strength in case said at least one caregiving person with mentioned authorization leaves said reading space V around/beside said patient monitor 10. Normally the restoration is operative when the caregiving person leaves the reading space without switching off the alarm through the operator interface 7. This function is not available if the reading spaces V within the patient monitor, i.e. a card slot, because the person can go away from the patient without a logging-out from the system, i.e. without repeated use of the badge in the slot.

In the preferred embodiment, where the data transfer unit is a radio transmitter and the badge comprises a passive RFID tag, the detection of the caregiving persons by said data transfer unit 8 comprises the main steps: initiating tag polling at least during said activated alarm; comparing the ID-data found in said reading space V with data of caregiving persons' authorizations, and logging-in the person(s) in question; and forwarding the authorization(s) into said program and/or logics as a variable. Here the system memory 5b or 5c is utilized to authenticate the caregiving person. If the authentication, like authorization, is included in the tag memory the detection of the caregiving persons by said data transfer unit 8 is more simple, comprising the main steps: initiating tag polling at least during said activated alarm; and founding data of caregiving persons' or persons' authorization(s) in said reading space V, logging-in the person(s) in question, and forwarding the authorization(s) into said program and/or logics as a variable.

Above it has been described how patient monitor alarm can be controlled by reading ID-data. The system can have further features that use the same identification of persons, because nowadays very often the memory or memories 5b, 5c also store(s) medical data of the patients 2. This patient data can be transferred to the patient monitor 10 so that it can be utilized by the caregiving person or persons present during alarming. The patient data is available through the data connection 9 from the data network 19 of the facility, and the data transfer is controlled by the processor 15. The transfer of the medical data of the patient into whom said sensor(s) 3a, 3b, 3c... is/are attached is allowed from said network memory or memories to be shown on the display 16 of the patient monitor 10 after logging-in by the caregiving person(s). In many cases the medical data allowable to be shown on the display 16 of the patient monitor is selected according to the caregiving person's authorization detected by said data transfer unit 8.

Typically the alarm is strength and/or a wavelength of a sound signal, i.e. audible signal, or a light signal, i.e. visual signal. The subduing of the alarm is a change of said strength and/or said wavelength. The intention is that alarm is more noticeable or annoying than the state of subdued alarm. Preferably the alarm is indicated by stronger alarm sound or a stronger alarm light, which can be also flashing, whereupon said subdued alarm is a weaker sound or light, or lack of alarm sound or alarm light - the latter meaning that the alarm is extinguished. Alternatively, the alarm can be indicated by special alarm sound or a special alarm color of the light, and subdued alarm is another kind of sound or another color of the light.

In case the facility like hospital has the data network 19 and a central control room said alarm can be also forwarded to this central control room of the facility, so that the whole system and all patients are under control. The forwarded alarm is then subdued in the central control room in a similar or different way analogous to the subduing of the patient monitor. Very often the alarm in the control room is some visual signal in a computer display, and subduing is another kind of visual signal or extinguishing the alarm.

## Claims

1. A system for electronic alarm control of patient monitors (10) in the presence of caregiving person or persons, comprising:
- each caregiving person (1) carries an identification badge (11);
- said identification badge includes at least a tag (4) storing a personal ID-data (D_{c}) in a tag memory (5a) in said badge, or at least a marking (24) with a personal ID-data (D_{c}) on said badge;
- said patient monitor (10) comprises:
- sensors (3a, 3b, 3c...) attached to a patient (2) and measuring medical data (D_{M}) from the patient,
- a processor (15), a display (16), and an operator interface (7) with an alarming unit (17),
- a data transfer unit (8) capable to read the data contents of said personal tags or badges within a limited reading space (V) inside or around or beside said patient monitor,
- said sensors, said operator interface, and said data transfer unit are connected to said processor, which is operating with a predetermined program and/or logics;
- said alarming unit (17) being controlled by said processor (15) so that:
- an alarm is activated when said medical data (D_{M}) measured from the patient deviates from a preset value or values or range of values,
- the activated alarm is subdued when at least one of said caregiving person badges enters said reading space (V) within/around/beside said patient monitor, and is detected by said data transfer unit using the data from the tag memory (5a) or onfrom the badge (11).

2. A system for electronic alarm control of patient monitors (10) in the presence of caregiving person or persons comprising:
- each caregiving person (1) carries an identification badge (11);
- said identification badge includes at least a tag (4) storing a personal ID-data (D_{C}) in a tag memory (5a) in said badge, or at least a marking (24) with a personal ID-data (D_{C}) on said badge;
- said patient monitor (10) comprises:
- sensors (3a, 3b, 3c...) attached to a patient (2) and measuring medical data (D_{M}) from the patient,
- a processor (15), a display (16), and an operator interface (7) with an alarming unit (17),
- a system memory (5b), in which at least said ID-data is stored, or a data connection (9) to a data network (19) of the facility in question, which network comprises a system memory (5c) storing at least said ID-data,
- a data transfer unit (8) capable to read the data contents of said personal tags or badged within a limited reading space (V) inside or around or beside said patient monitor,
- said sensors, said memory, said operator interface, and said data transfer unit are connected to said processor, which is operating with a predetermined program and/or logics;
- said alarming unit (17) being controlled by said processor (15) so that:
- an alarm is activated when said medical data (D_{M}) measured from the patient deviates from a preset value or values or range of values,
- the activated alarm is subdued when at least one of said caregiving person badges enters said reading space (V) within/around/beside said patient monitor, and is detected by said data transfer unit using the data from the tag memory (5a) or onfrom the badge (11), and data from the system memory (5b or 5c).

3. A system according to claim 1 or 2, wherein said data transfer unit (8) is a short-range radio transmitter (18) in the patient monitor (10) with an antenna (6a) or set of antennas (6b, 6c) arranged to have a limited reading space (V) around or beside said patient monitor, and said tag (4) is a passive RFID tag with a tag memory (5a); and that personal ID-data (D_{C}) is a byte or bytes stored in said tag memory.

4. A system according to claim 1 or 2, wherein said data transfer unit (8) is a receiver (34) in the patient monitor (10) together with a RF-transmitter or IR-transmitter (38) in the badge (11) producing a limited reading space (V) around or beside said patient monitor, and said tag (4) is an active RFID tag or IR tag with a tag memory (5a); and that personal ID-data (D_{C}) is a byte or bytes stored in said tag memory.

5. A system according to claim 1 or 2, wherein said data transfer unit (8) is an optical or magnetic or electrical card reader (28) in the patient monitor (10); and that the personal ID-data (D_{c}) marking (24) is a line code, or set of characters and/or numbers printed on the badge (11), or a byte or bytes stored in said tag memory.

6. A system according to any of the claims 1 to 4, wherein the activated alarm is restored to the initial value in case all but one of caregiving person badges leave, without switching off the alarm through the operator interface (7), said reading space (V) around/beside said patient monitor (10), thereby becoming non-detected by said data transfer unit (8).

7. A system according to any of the preceding claims, wherein said program and/or logics of the processor (15) is such that it allows the activated alarm to be switched off through the operator interface (7) only by a detected caregiving person.

8. A system according to any of the claims 2 to 7, wherein the ID-data (D_{C}) is considered to belong to a caregiving person, when the ID-data is readable by the data transfer unit (8).

9. A system according to claim 8, wherein the ID-data (D_{C}) is considered to belong to a caregiving person, when the ID-data contains a further data indicating the fact that the badge belong to a member of the nursing staff.

10. A system according to any of the claims 1 or 3 to 7, wherein the ID-data (D_{c}) is considered to belong to a caregiving person, when the ID-data is found from the system memory (5b or 5c) as such.

11. A system according to any of the preceding claims, wherein each caregiving person (1) of said nursing staff have a predetermined competence and authorization to perform specified medical operation or operations; that the carried identification badge (11) have on it or have in its memory, or the system memory has said competence/authorization data, which enable him/her to perform the actions necessitated by the measured medical data having caused said alarm.

12. A system according to any of the claims 2 to 3, or 5 to 11, wherein said detection of the caregiving persons by said data transfer unit (8) comprises:
- initiating tag polling at least during said activated alarm;
- comparing the ID-data found in said reading space (V) with data of caregiving persons' authorizations, and logging-in the person(s) in question; and
- forwarding the authorization(s) into said program and/or logics as a variable.

13. A system according to any of the claims 1, 3, or 5 to 11, wherein said detection of the caregiving persons by said data transfer unit (8) comprises:
- initiating tag polling at least during said activated alarm; and
- founding data of caregiving persons' or persons' authorization(s) in said reading space (V), logging-in the person(s) in question, and forwarding the authorization(s) into said program and/or logics as a variable.

14. A system according to claim 12 or 13, wherein said program and/or logics produces, according to the authorization(s):
- said subduing of the alarm and allowance of the alarm switch off, or
- restoration of the initial value of the alarm.

15. A system according to any of the claims 1 to 4, or 6 to 14 , wherein said limited reading space (V) around the patient monitor (10) is attained by antenna (6a) configuration and/or antenna (6b, 6c) positioning, and by low energy RF-field (F).

16. A system according to any of the preceding claims, wherein said system memory or memories (5b, 5c) also store(s) medical data of the patients (2); and that said data connection to the data network (19) of the facility is controlled by said processor (15) so that a transfer of the medical data of the patient into whom said sensor(s) (3a, 3b, 3c...) is/are attached is allowed from said network memory or memories to be shown on the display (16) of the patient monitor (10).

17. A system according to any of claims 11 to 16, wherein said medical data allowable to be shown on the display (16) of the patient monitor is selected according to the caregiving person's authorization detected by said data transfer unit.

18. A system according to any of the claims 11 to 16, wherein said program and/or logics of the processor (15) is such that it allows the activated alarm to be switched off through the operator interface (7) only by a detected caregiving person with such authorization that enable him/her to perform the actions necessitated by the measured medical data having caused said alarm.

19. A system according to any of the preceding claims, wherein said data transfer unit (8) is arranged to be capable to read the data contents from several tags or badges, and said alarming unit (17) is controlled by said processor (15) to enable further steps on the basis of these several tags or badges.

20. A system according to any of the preceding claims, wherein said alarm is a strength and/or wavelength of sound signal or light signal; and that said subduing is a change of said strength and/or said wavelength.

21. A system according to any of the preceding claims, wherein said alarm is forwarded to a central control room of the facility, and said forwarded alarm is subdued in the central control room in a similar or different way analogous to the subduing of the patient monitor.
